# EUROPÄISCHE PATENTSCHRIFT

(11) **EP 1 449 815 B1**
(45) Veröffentlichungstag und Bekanntmachung des Hinweises auf die Patenterteilung: **01.07.2009**
(21) Anmeldenummer: 04003768.1
(22) Anmeldetag: 19.02.2004
(51) Int. Cl.: C03C 10/02, C03C 4/00, A61L 27/00

(54) **Bioaktive Rhenanit-Glaskeramik**
Bioactive rhenanite glass-ceramics
Vitrocéramique bioactive comprenant rhenanite

(30) Priorität: 21.02.2003 DE 10307646
(43) Veröffentlichungstag der Anmeldung: 25.08.2004
(73) Patentinhaber: Ivoclar Vivadent AG, 9494 Schaan (LI)
(72) Erfinder: Apel, Elke, CH-9475 Sevelen (CH); Höland, Wolfram, FL-9494 Schaan (LI); Rheinberger, Volker, FL-9490 Vaduz (LI)
(74) Vertreter: UEXKÜLL & STOLBERG

(56) Entgegenhaltungen:
- WO-A-91/07357
- WO-A-93/17976
- GONG WEILIANG ET AL: "Porous bioactive glass and glass-ceramics made by reaction sintering under pressure" J BIOMED MATER RES; JOURNAL OF BIOMEDICAL MATERIALS RESEARCH MAR 2001 JOHN WILEY & SONS INC, NEW YORK, NY, USA, Bd. 54, Nr. 3, März 2001 (2001-03), Seiten 320-327, XP001193745
- DATABASE CHEMABS CHEMICAL ABSTRACTS SERVICE, COLUMBUS, OHIO, US; Bd. 58, Nr. 11, Zf. 11724 27. Mai 1963 (1963-05-27), KANAGAWA: "Fusion products of the lime-soda-phosphorus pentoxide-silica system" XP002284173 Database accession no. 58:11724 & KOGYO KAGAKU ZASSHI, Bd. 65, 1962, Seiten 872-877,

## Beschreibung

Die Erfindung betrifft eine bioaktive rhenanithaltige Glaskeramik, die sich durch einen hohen Gehalt an P₂O₅, CaO und Na₂O auszeichnet und sich insbesondere als Knochenersatzmaterial auf dem Dentalgebiet oder Ersatzmaterial für Dentin oder Zahnschmelz, d.h. als Restaurationsmaterial, eignet.

Aus dem Stand der Technik sind bioaktive Gläser und Glaskeramiken bekannt. Es handelt sich hierbei einerseits um Materialien, die auf einem silicatischen Grundsystem beruhen und P₂O₅ als Zusatzkomponente aufweisen und andererseits um die Gruppe der SiO₂-freien Phosphatgläser- und -glaskeramiken. Kennzeichnend für diese Materialien sind das Vorhandensein einer glasig-amorphen Grundmatrix und im Falle der Glaskeramiken zusätzlich einer oder mehrerer kristalliner Phasen.

Darüber hinaus gibt es die Gruppe der kristallinen, glasfreien Phosphate, die als Ein- oder Mehrstoffsysteme Einsatz als Knochenersatzmaterial finden und über eine ausgezeichnete Resorbierbarkeit verfügen.

Allen diesen bekannten Werkstoffgruppen liegt jeweils nur ein Mechanismus der Oberflächenreaktivität zugrunde, wie die folgenden Erläuterungen zeigen.

Die bioaktiven, SiO₂-haltigen Glaskeramiken für den Knochenersatz besitzen hauptsächlich Apatitkristalle als wesentliche Phase zur Förderung der Regeneration des lebenden Knochens. Sie werden für den Knochenersatz in der Humanmedizin bzw. der Zahnmedizin in der Weise verwendet, dass sie eine reaktive Oberflächenschicht liefern, die ihrerseits die zusätzliche Bildung von Apatitkristallen fördert. Zu dieser Gruppe der apatithaltigen silicatischen Glaskeramiken zählen z.B.
- Cerabone^{®}: Apatit - Wollastonit - Glaskeramik
- Ceravital^{®}: Apatit - Glaskeramik
- Bioverit II^{®}: Apatit - Glimmer - Glaskeramik.

Bioaktive Gläser, also Werkstoffe ohne Kristalle, sind ebenfalls bekannt. Bioglass^{®} und PerioGlas^{®} sind Vertreter des Stoffsystems Na₂O-Cao-SiO₂-P₂O₅. Hench (J. Am. Ceram. Soc., 74 (1991) 1487-1510) beschreibt das direkte Verwachsen zwischen derartigen Gläsern mit dem natürlichen Knochen in 11 Reaktionsstufen. Für diese Materialien ist eine schnelle Oberflächenreaktion typisch, die mit der Freisetzung von Ca-, Na-, P- und anderen Ionen verbunden ist. Nachteilig wirkt sich diese hohe Löslichkeit jedoch auf die mechanische Festigkeit der bioaktiven Gläser aus. Der Einsatz als Knochenersatz mit lasttragenden Funktionen, z.B. für den Ersatz von Rückenwirbeln, ist im Unterschied zu den Apatit-Wollastonit-Glaskeramiken somit nicht möglich.

Ein weiterer Nachteil der bioaktiven Gläser besteht darin, dass die Granulatpartikel wegen ihrer hohen Löslichkeit in Körperflüssigkeiten vom Ort der Implantation und aus deren Umgebung wegtransportiert werden, noch ehe sich neuer Knochen bilden kann.

Peitl et al. (J. Noncryst. Solids, 292 (2001) 115-126) beschreibt eine auf Bioglass^{®} basierende Glaskeramik, die eine phosphatfreie kristalline Phase (NaCa₂Si₃O₉) und eine phosphathaltige Restglasmatrix umfasst. Sie weist gegenüber den genannten Bioglaskeramiken eine verbesserte Bioaktivität auf, und sie führt in einer der menschlichen Körperflüssigkeit nachempfundenen Lösung, Simulated Body Fluid (SBF), zur Bildung von Hydroxyl-Carbonatapatit (HCA).

Aus der DE-A-41 13 021 ist mit Bioverit^{®} III weiterhin eine vollständig resorbierbare SiO₂-freie Glaskeramik bekannt. Sie besitzt als Hauptkristallphasen Apatit und AlPO₄ sowie weitere komplexe Phosphate. Sie weist jedoch den schwerwiegenden Nachteil auf, dass sie Al³⁺-Ionen enthält, die für das biologische System der Knochenregeneration störend sind. Dies führt dazu, dass ab einer bestimmten Konzentration die Bildung von Apatit erschwert oder gar komplett verhindert wird.

In der WO 01/12242 sind vollständig glasfreie, kristalline Phosphatverbindungen, wie z.B. Ca₂NaK(PO₄)₂ und CaKPO₄, ebenfalls als Biowerkstoffe beschrieben. Diese Natriumkalium- oder Kalium-Calcium-Phosphate verbessern die biologische Abbaubarkeit und die Knochenneubildung. Ca₂KNa(PO₄)₂ ist sehr gut geeignet, um Knochendefekte auszuheilen, wie von Niu Jinlong et al. (J. Mat. Science 36 (2001) 3805-3808) und G. Berger et al. (Biomaterials 16 (1995) 1241-1258) beschrieben wurde. Diese kristallinen Werkstoffe besitzen jedoch den Nachteil, dass sie eine sehr hohe Zersetzungs- und Umwandlungsrate besitzen. Dadurch sind die Lagerstabilität dieser Werkstoffe und auch die Verarbeitungszeit während der klinischen Applikation extrem kurz. Auch die Möglichkeiten der Formgebung sind gegenüber Gläsern und Glaskeramiken eingeschränkt.

Rhenanit oder Rhenanit in Kombination mit anderen bekannten Kristallphasen, besonders dem Hydroxylapatit, ist ebenfalls bereits als Knochenersatzmaterial beschrieben worden.

Die bioaktiven Eigenschaften von reinem ß-NaCaPO₄, d.h. Rhenanit, wurden in Arbeiten von Driessens et al. (J. Mat. Science, 3 (1992) 413-417) und Suchanek et al. (J. Europ. Cer. Soc. 18 (1998) 1923-1929) beschrieben. Danach zeigt Rhenanit in SBF Osteokonduktivität, d.h. ein hohes Mass an Effizienz und Qualität als Knochenersatzmaterial.

In Calciumphosphatgläsern und -glaskeramiken tritt Rhenanit neben Apatit und weiteren kristallinen Calciumphosphatphasen als Nebenphase auf. Zhang et al. (J. Non-Cryst. Solids 272 (2000) 14-21) beschreiben die Bildung von Rhenanit in einem phosphathaltigen, SiO₂-freien Grundsystem unter Zugabe von maximal 5 Mol% Na₂O.

Weiterhin wird Rhenanit in der DE-A-197 25 555 als mögliche Nebenkristallphase in einer transluzenten Apatit-Leucit-Glaskeramik beschrieben, die als Verblendmaterial für keramische Dentalrestaurationen verwendet wird. Diese Glaskeramik ist jedoch nicht oberflächenreaktiv, d.h. nicht bioaktiv und sie enthält Al₂O₃, welches für das biologische System nachteilig ist.

Weiterhin werden bioaktive ,glaskeramikartige Produkte in Arbeiten von Gong W. et al. (Journal of Biomedical Materials Research, vol. 54 , no. 3 (2001) Seite 320-327) beschrieben. In diesen kristallinen Produkten wird das Ausgangsapatit in Rheanit umgesetzt.

Der Erfindung liegt demgemäß die Aufgabe zugrunde, eine bioaktive Glaskeramik zur Verfügung zu stellen, die sich im Unterschied zu anderen SiO₂-haltigen Systemen insbesondere durch eine regulierbare Reaktionskinetik zur Bildung von Apatit in SBF auf der Oberfläche des Werkstoffes auszeichnet. Weiterhin soll die Glaskeramik auch ohne Gehalte an biologisch nachteiligen Substanzen wie Al-Verbindungen erzeugt werden können.

Diese Aufgabe wird überraschenderweise durch die bioaktive Rhenanit-Glaskeramik nach den Ansprüchen 1 bis 12 gelöst.

Gegenstand der Erfindung sind ausserdem das Verfahren nach Anspruch 13, Formkörper nach den Ansprüchen 14 und 15, die Verwendung gemäß Anspruch 16 sowie das bioaktive Kompositmaterial nach Anspruch 17.

Die erfindungsgemäße bioaktive Rhenanit-Glaskeramik mit kristallliner Phase und Glasphase ist **dadurch gekennzeichnet, dass** die kristalline Phase Rhenanit enthält und die Glaskeramik die folgenden Komponenten enthält:

| Komponenten | Anteil (Gew.-%) |
|---|---|
| SiO₂ | 29,5 bis 70,0 |
| CaO | 5,5 bis 23,0 |
| Na₂O | 6,0 bis 27,5 |
| P₂O₅ | 2,0 bis 23,5 |
| F | 0 bis 1,5 |

und im Wesentlichen frei von Al₂O₃ ist.

Vorzugsweise bildet bei den erfindungsgemäßen Glaskeramiken Rhenanit, d.h. β-NaCaPO₄, die Hauptkristallphase. Die bioaktive Restglasphase trägt durch ihre hohe Löslichkeit in SBF zu einer schnellen Apatitbildung bei, während die osteokonduktiv wirkende Rhenanit-Kristallphase zu einer langsameren Apatitbildung führt, aber eine verbesserte mechanische Stabilität eines Implantats gewährleistet. Dieser Doppelmechanismus der Reaktivität der Glaskeramik ermöglicht die Steuerung der Bioaktivität und ist ein besonderer Vorzug der erfindungsgemäßen Glaskeramik.

Vorzugsweise enthält die erfindungsgemäße Glaskeramik weniger als 0,1 Gew.-% Al₂O₃, besonders bevorzugt weniger als 0,01 Gew.-% Al₂O₃ und ganz besonders bevorzugt ist sie frei von Al₂O₃. Al₂O₃ könnte, wie oben beschrieben, die Bioaktivität der Glaskeramik und die Bildung von Apatit verringern.

Die Löslichkeit der Glaskeramik ist gegenüber den oben beschriebenen, bekannten Biogläsern geringer. Durch diese herabgesetzte Löslichkeit wird vermieden, dass sie vom Ort der Implantation wegtransportiert wird, noch ehe sich neuer Knochen bilden kann. Zudem sind auch die Zersetzungs- und Umwandlungsrate nicht so hoch wie bei den glasfreien Phosphaten, bei denen die Verarbeitungszeit und die Lagerstabilität der biokompatiblen Materialien durch den schnellen reaktionskinetischen Stoffumsatz zur Apatitbildung eingeschränkt sind. Durch die Variation des kristallinen Anteils an Rhenanit in der Glaskeramik ist eine gezielte Steuerung der Reaktionskinetik der Apatitbildung möglich, und es sind Glaskeramiken bevorzugt, die zwischen 4 und 50 Gew.-%, bevorzugt zwischen 10 und 50 Gew-% oder zwischen 4 und 40 Gew.-% Rhenanit enthalten.

Erfindungsgemäß bevorzugt ist eine Glaskeramik, die die folgenden Komponenten unabhängig voneinander in folgenden Mengen enthält:

| Komponenten | Anteil (Gew.-%) |
|---|---|
| SiO₂ | 29,5 bis 65,5 |
| CaO | 6,0 bis 23,0 |
| | (insbesondere 11,0 bis 23,0) |
| Na₂O | 7,0 bis 25,5 |
| P₂O₅ | 3,0 bis 23,5 |
| | (insbesondere 5,5 bis 23,5) |
| F | 0,5 bis 1,2 . |

Der Begriff "unabhängig voneinander" bedeutet, daß auch nur wenigstens einer der bevorzugten Mengenbereiche gewählt werden kann.

Ganz besonders bevorzugt ist eine Glaskeramik, die die folgenden Komponenten unabhängig voneinander in den folgenden Mengen enthält:

| Komponenten | Anteil (Gew.-%) |
|---|---|
| SiO₂ | 35,0 bis 60,0 |
| CaO | 15,0 bis 23,0 |
| Na₂O | 9,0 bis 25,5 |
| | (insbesondere 7,0 bis 18,0) |
| P₂O₅ | 10,0 bis 23,5 |
| | (insbesondere 10,0 bis 20,0) |
| F | 0, 5 bis 1,2 |

Eine solche Rhenanit-Glaskeramik zeichnet sich insbesondere durch eine hohe Oberflächenreaktion zur Bildung von Phosphaten, insbesondere Apatit, in SBF aus.

Es ist vorteilhaft, wenn die Glaskeramik zur gezielten Beeinflußung der nachstehenden Eigenschaften mindestens eine der folgenden Zusatz-Komponenten enthält, die von den vorstehend genannten Komponenten verschieden sind.

Zur Verbesserung der biologischen Reaktionsverhaltens haben sich Zusätze von Oxiden von K, B, Ti, Zr, Nb, Ta bewährt. Dabei bilden sich an den Oxiden der Elemente Ti, Zr, Nb und Ta reaktive OH-Gruppen. Die Oxide von B haben grundsätzlich eine hohe Bioverträglichkeit. K wird anstelle des Na in den Rhenanit eingebaut und verstärkt über eine Mischkristallbildung die Kristallphase insgesamt.

Zur Erhöhung der Röntgenopazität haben sich Zusätze von Oxiden der Elemente Nb, Ta, Y und La bewährt.

Eine antimikrobielle Wirkung wird durch den Zusatz von Ag, Zn und I zu der Glaskeramik erreicht.

| Zusatz-Komponenten | Anteil (Gew.-%) |
|---|---|
| R^{(I)}₂O | 0 bis 15,0 |
| R^{(II)}O | 0 bis 4, 0 |
| R^{(III)}₂O₃ | 0 bis 10,0 |
| R^{(IV)}O₂ | 0 bis 10, 0 insbesondere bis 1,0 |
| Hal | 0 bis 2,0 |

wobei
R^{(I)} für ein einwertiges Kation, insbesondere K oder Ag
R^{(II)} für ein zweiwertiges Kation, insbesondere Zn
R^{(III)} für ein dreiwertiges Kation, insbesondere B, Nb,
Ta, Y, La oder ein Lanthanid,
R^{(IV)} für ein vierwertiges Kation, insbesondere Ti und
Hal für ein Halogenidion, insbesondere Br oder I steht.

Sofern Zusatz-Komponenten vorhanden sind, beträgt ihr Anteil an der Glaskeramik mindestens 0,1 Gew.-%.

Besonders bevorzugt sind Glaskeramiken, die aus den vorstehend angegebenen Komponenten und gegebenenfalls den erwähnten Zusatz-Komponenten bestehen.

In der erfindungsgemäßen Glaskeramik ist es weiterhin vorteilhaft, wenn das Gewichtsverhältnis von Na₂O : CaO von 0,8 bis 2,0 und das Gewichtsverhältnis von CaO : P₂O₅ von 0,9 bis 2,2 beträgt. Dies ist insbesondere vorteilhaft, wenn spontan kristallisierende Glaskeramiken erzeugt werden sollen.

In der erfindungsgemäßen Glaskeramik ist es auch vorteilhaft, wenn das Gewichtsverhältnis von Na₂O : CaO von 1,0 bis 2,1 und das Gewichtsverhältnis von CaO : P₂O₅ von 0,9 bis 2,2 beträgt. Dies ist insbesondere dann vorteilhaft, wenn Glaskeramiken hergestellt werden sollen, zu deren Erzeugung eine Wärmebehandlung erforderlich ist.

Es ist ausserdem vorteilhaft, wenn die kristalline Phase der erfindungsgemäßen Glaskeramik zusätzlich mindestens eine der folgenden kristallinen Komponenten enthält: Natrium-Calcium-Silicat, Apatit, Natrium-Phosphat, Natrium-Calcium-Phosphat und Natrium-Kalium-Calcium-Phosphat.

Bevorzugt ist eine Rhenanit-Glaskeramik, bei der die Rhenanit-Kristalle im Maximum 10 µm groß sind. Weiterhin ist eine Rhenanit-Glaskeramik bevorzugt, bei der die Rhenanit-Kristalle eine mittlere Größe (Zahlenmittel) zwischen 0,01 und 5,0 µm haben. Ganz besonders bevorzugt liegt die mittlere Größe zwischen 0,15 und 2,5 µm, da hierbei durch Resorptionsprozesse die Bioaktivität positiv beeinflusst wird. In dieser bevorzugten Größtenordnung wird durch die Reaktionskinetik des Auflösens und Umwandelns der Rhenanit-Kristalle das Einwachsen eines Implantats besonders erleichtert. Dabei ist es gelungen, durch die Kristallisation im Nanobereich Kristalle in der mittleren Größe (Zahlenmittel) von 0,01 und 5,0 µm zu erzeugen, wobei durch Verfahrensführung die Ausbildung von isoliert in der Restglasmatrix vorliegenden Kristallen oder von Agglomeraten erzielt werden kann, indem neue Kristalle auf der Oberfläche von bereits entstandenen als eigenständige Kristalle aufwachsen und nicht primär die Kristallgröße der bereits gebildeten vergrößern.

Zur Herstellung der erfindungsgemäßen Glaskeramik wird
a) ein Ausgangsglas mit einer Zusammensetzung, das die Komponenten und gegebenenfalls Zusatzkomponenten enthält, bei Temperaturen von 1200 °C bis 1650 °C erschmolzen,
b) die Glasschmelze aus a) abgekühlt, insbesondere die Glasschmelze
   (i) in Wasser gegossen, wobei sich ein Glasgranulat bildet oder
   (ii) in eine Form gegossen oder
   (iii) zwischen Metallplatten abgeschreckt,
c) gegebenenfalls das abgekühlte Glas aus b) bei Temperaturen von 600 bis 1000, insbesondere 600 °C bis 980 °C für eine Dauer von 10 Minuten bis zu 10 Stunden, insbesondere bis zu 8 Stunden wärmebehandelt und
d) gegebenenfalls die Glaskeramik, die aus b) oder c) resultiert, zu einem Pulver mit einer Korngröße von 100 nm bis 100 µm, insbesondere 1 bis 50 µm zerkleinert.

Bei höheren Phosphatgehalten, ab 6 Gew.-% P₂O₅, wird das Ausgangsglas zweckmäßigerweise zunächst in einem Al-freien Sinter-Tiegel in ungefähr einer Stunde von Raumtemperatur auf 1200 °C erhitzt und anschliessend abgekühlt. Dieser so entstandene Sinterkuchen wird dann an Stelle des Ausgangsglases, wie oben beschrieben, im Pt-Rh-Tiegel bei 1200 bis 1650 °C geschmolzen.

Bei der Herstellung der erfindungsgemäßen Glaskeramik kann der Rhenanit sowohl durch nachträgliche Kristallisation mittels der Wärmebehandlung des Ausgangsglases in Stufe (c) als auch durch spontane Kristallisation beim Abkühlen des Glases aus der Schmelze in Stufe (b) entstehen. Die Kristallisation des Rhenanit wird dabei maßgeblich durch das Verhältnis Na₂O : CaO und den Phosphatgehalt in der Zusammensetzung bestimmt.

Bei der spontanen Kristallisation kann der Rhenanit als alleinige Kristallphase auftreten oder auch, abhängig von den Zusammensetzungen, gemeinsam mit Silikaten (u.a. Na₂SiO₅, Na₂CaSi₃O₈, NaCa₂Si₃O₉, NaCa₃Si₆O₁₆) oder weiteren Phosphaten wie (Na₃PO₄, Na₂Ca(PO₄)F, KNaCa₂(PO₄)₂, KCa(PO)₄, oder Ca₅(PO₄)₃F).

Aus Ausgangsgläsern mit hoher Neigung zur Phasentrennung kristallisiert bei Wärmebehandlung Rhenanit aus den Entmischungstropfen aus. Die Kristalle sind dabei im Zahlenmittel vorzugsweise zwischen 0,01 und 0,5 µm groß. Die Kristallphase hat dabei insbesondere einen Anteil von 4 bis 50 Gew.-%. Längere Temperzeiten oder höhere Temperaturen führen zu gröberen Strukturen. Die zunächst einzelnen Tropfen schließen sich paarweise zusammen und lagern sich kettenförmig aneinander. Die bevorzugte Wärmebehandlung in Stufe (c) erfolgt für Zusammensetzungen mit niedrigen Phosphatgehalten im Temperaturbereich von 650 bis 750 °C mit Haltezeiten von 1 bis 6 Stunden und für Zusammensetzungen mit Nanokristall-Ausscheidungen im Temperaturbereich von 650 bis 1000 °C in ein-oder mehrstufigen Prozessen der thermischen Behandlung für 0,5 bis 10 Stunden.

Bei spontaner Kristallisation bilden sich größere Schwärme mit verzweigten Kristallgefügen. In diesem Fall entsteht bevorzugt ein Verbund- oder Durchdringungsgefüge, wobei gröbere Kristalle mit 0,2 bis 0,8 µm Dicke und etwa 1,0 bis 5,0 µm Länge vorliegen.

Je nach Zusammensetzung und Abkühlbedingungen entstehen aus der Schmelze extrem feine Kristallite (0,05 µm Durchmesser), die somit ein nanokristallines Gefüge aufweisen.

Der kristalline Anteil aller Typen der erfindungsgemässen Glaskeramiken beträgt etwa 4 bis etwa 50 Gew.-%.

Formkörper, die die erfindungsgemäße Glaskeramik enthalten und insbesondere aus ihr bestehen sind ein weiterer Bestandteil der vorliegenden Erfindung. Diese Formkörper sind bevorzugt monolithisch, also dichte oder poröse Körper mit offenen Poren mit einem Porendurchmesser von ungefähr 2 bis 200 µm. Es kann sich hierbei insbesondere um Knochenimplantate, insbesondere für den dentalen Bereich handeln.

Die Erfindung betrifft ausserdem ein Verfahren zur Herstellung eines Formkörpers aus der erfindungsgemäßen Glaskeramik, bei dem zunächst die erfindungsgemäße Glaskeramik in gewünschter Weise verformt und der erhaltene Formkörper nach einem Pressen für eine Dauer von ungefähr 30 Minuten bei 600 °C bis 750 °C gesintert wird.

Zur Herstellung von Formkörpern, ist es ebenso möglich ein Ausgangsglas mit einer Zusammensetzung, das die oben genannten Komponenten der Rhenanit-Glaskeramik und gegebenenfalls die oben genannten Zusatz-Komponenten enthält, auf die gewünschte Korngröße mit gewünschter Korngrößenverteilung aufzumahlen, wobei ein Zahlenmittel der Korngröße von 0,1 bis 50 µm bevorzugt ist und die Korngrößenverteilung mono-, bi- oder trimodal sein kann, und aus den Pulvern Presslinge herzustellen, die dann in einem vorgegebenen Zeit- und Temperaturintervall, z.B. 30 Minuten bei 600 °C, zu dichten Formkörpern gesintert werden. Durch diese Wärmebehandlung kann dann die Kristallisation des Glases und damit die Bildung der Glaskeramik bewirkt werden.

Ein offenporiger Formkörper lässt sich nach dem von Kim et al. in Biomaterials 24, 3277 - 3284, 2003 beschriebenen Verfahren herstellen, indem die innere Oberfläche der Poren eines Kunststoffschwammes, z.B. eines Polyurethan-Schwammes, mit einem dünnflüssigen Schlicker mit einer Korngrösse der Glaskeramikteilchen von kleiner als 300 µm beschichtet wird. Anschliessend wird der Kunststoffschwamm bei langsamer Aufheizrate bis ca. 700 °C ausgebrannt. Das Ergebnis ist ein Keramikgerüst ("scaffold") mit offenen Poren von etwa 100 bis 200 µm Durchmesser.

Die Verwendung der erfindungsgemäßen Glaskeramik als Material zum Wiederaufbau von Knochen und/oder zum Ersatz von Knochen, insbesondere im dentalen Bereich, d.h. als Dentalmaterial, oder als Ersatzmaterial für Zahnsubstanz, wie insbesondere von Dentin oder Zahnschmelz ist ebenfalls Bestandteil der Erfindung.

Bevorzugt wird die erfindungsgemäße Glaskeramik zur Förderung des Knochenwachstums, insbesondere im dentalen Bereich, eingesetzt. Sie kann auch z.B. sehr vorteilhaft in der Umgebung von Metallimplantaten, als bioaktive Schicht von biokompatiblen Materialien, zum Pulpenschutz, als Dentin- und Schmelzersatz in Form eines Zusatzes zu einem Zahnpflegemittel wie einer Zahnpasta, als Gerüst- und/oder als Trägermaterial für bioaktive Wirkstoffe, wie z.B. Wachstumsfaktoren, Hormone, Proteine, Polypeptide, Saccharide u.a. oder als Werkstoff mit bakteriostatischer oder therapeutischer Wirkung mit kontrollierter Ionenfreisetzung und/oder Wirkstoffabgabe verwendet werden.

Die erfindungsgemässe Glaskeramik kann überdies in Kombination mit organischen Verbindungen verwendet werden. Sie wird dabei insbesondere in monolithischer Form, Pulverform oder poröser Form eingesetzt. Die organischen Verbindungen können Biopolymere, die auf Hydroxysäuren oder cyclischen Carbonaten basieren, Lactate und/oder Acrylamide sein. Derartige Kombinationen sind als bioaktive Kompositwerkstoffe insbesondere im Dentalbereich einsetzbar. Die Erfindung betrifft daher auch solche bioaktiven Kompositwerkstoffe.

Die Erfindung wird im Folgenden durch Beispiele weiter erläutert:

### Beispiele:

Es wurden 15 verschiedene Gläser des Systems SiO₂-Na₂O-CaO-P₂O₅ hergestellt und je nach Zusammensetzung entweder nach Abkühlung der Schmelze gesteuert kristallisiert oder direkt während des Abkühlvorganges kristallisiert. Die Zusammensetzungen der Gläser sind in Tabelle 1 angegeben. Alle Proben wurden als 120 g - Batche im Pt/Rh-Tiegel bei Temperaturen von 1200 °C bis 1650 °C geschmolzen. Je nach Zusammensetzung betrug die Schmelzdauer 1 bis 3 Stunden. Aus der Schmelze wurden Stäbe mit 11 mm Durchmesser und 55 mm Länge in vorgewärmte Stahlformen gegossen.

**Tabelle 1:**

| Komponenten in Gew.-% | | | | | | |
|---|---|---|---|---|---|---|
| Nr. | SiO₂ | CaO | Na₂O | P₂O₅ | F | K₂O |
| 1 | 62,4 | 12,9 | 14,6 | 10,1 | 0 | 0 |
| 2 | 70,0 | 8,0 | 16,0 | 5,5 | 0,5 | 0 |
| 3 | 69,3 | 16,2 | 6,2 | 7,5 | 0,8 | 0 |
| 4 | 60,4 | 5,5 | 26,9 | 6,5 | 0,7 | 0 |
| 5 | 29,8 | 21,1 | 25,4 | 23,1 | 0,6 | 0 |
| 6 | 55,0 | 12,5 | 25,0 | 6,3 | 1,2 | 0 |
| 7 | 39,8 | 22,7 | 27,3 | 9,6 | 0,6 | 0 |
| 8 | 55,8 | 12,4 | 25,0 | 6,3 | 0,5 | 0 |
| 9 | 59,3 | 13,7 | 20,1 | 6,3 | 0,6 | 0 |
| 10 | 45,2 | 22,1 | 22,1 | 10,0 | 0,6 | 0 |
| 11 | 65,3 | 15,0 | 12,0 | 7,0 | 0,7 | 0 |
| 12 | 54,7 | 16,3 | 19,4 | 9,0 | 0,6 | 0 |
| 13 | 35,1 | 22,0 | 22,0 | 20,0 | 0,9 | 0 |
| 14 | 35,1 | 22,0 | 9,4 | 20,0 | 0,9 | 12,6 |
| 15 | 58,9 | 12,5 | 25,0 | 3,0 | 0,6 | 0 |

Bei den Gläsern nach den Beispielen 1, 2, 4 und 6 bis 10 und 15 kristallisiert der Rhenanit durch nachfolgende Wärmebehandlung bei 750 °C für 2 bis 8 Stunden und es bilden sich erfindungsgemäße Rhenanit-Glaskeramiken. Bei den Gläsern nach Beispiel 3, 5 und 11 bis 14 kristallisiert der Rhenanit nach dem Giessen in eine Form beim Abkühlen spontan aus der Schmelze unter Bildung erfindungsgemäßer Rhenanit-Glaskeramiken.

In Tabelle 2 ist für die Beispiele 8, 10, 13, 14 und 15 angegeben, unter welchen Bedingungen (Schmelztemperatur und - dauer) die jeweiligen Ausgangsgläser aufgeschmolzen wurden. Weiter gibt die Tabelle an, welches Aussehen die Materialien nach dem Abkühlen aufwiesen. Für die nicht spontan kristallisierenden Gläser der Beispiele 8 und 10 sind auch Angaben über die Wärmebehandlung gemacht. Schliesslich sind die jeweiligen Glaskeramiken in Bezug auf ihre Materialeigenschaften und dabei insbesondere ihre Kristallphasen und Rhenanitanteile sowie auf ihre Fähigkeit, die Bildung von Apatit in SBF zu bewirken, beschrieben.

Insbesondere für Beispiel 8 wurde ein Mikrogefüge mit homogen verteilten Kristalliten im nanoskaligen Bereich mit einer mittleren Kristallitgrösse von ca. 200 nm erzielt. Dies konnte durch eine thermische Behandlung bei 900 °C / 1 h erreicht werden. Im Beispiel 15 wurde durch eine gesteuerte Kristallisation bei 750 °C/ 8 h und 900 °C/ 1 h eine Rhenanit-Glaskeramik mit Nanokristallen einer Größe von ca. 20 bis 100 nm erzeugt. Mittels Differential-Thermoanalyse (Netzsch STA 409 PC) wurden die Transformationstemperaturen T_{g} und die Kristallisatibnstemperaturen bestimmt. Zur Messung wurde Glas- bzw. Glaskeramikgranulat mit einer Teilchengröße kleiner 90 µm verwendet. Für den Nachweis der erfolgten Kristallisation in Beispiel 15 wurden REM-Aufnahmen durchgeführt, da die DSC-Peaks für den geringen Kristallgehalt in der Glaskeramik zu klein oder die Messergebnisse recht ungenau wären.

Für die In-vitro-Tests zur Apatitbildung wurden jeweils zwei Massivplättchen von 11 mm Durchmesser und 2 mm Dicke präpariert und in 50 ml SBF in verschlossenen Polyethylenflaschen bei 37 °C gelagert. Die SBF-Lösung, auch als Kokubo-Lösung Nr. 9 bekannt, (siehe Kokubo et al. in J. Biomed. Mater. Res. 24 (1990) 721), wurde frisch hergestellt und bei 37°C mit 45 mM HCl und 50 mM (CH₂OH)₃CNH₂ auf einen pH-Wert von 7,3 eingestellt.

Nach jeweils 24 Stunden wurden die Probekörper mittels Rasterelektronenmikroskopie (REM) und energiedispersiver Röntgenspektroskopie (EDX) auf die Bildung von Apatit untersucht. Binnen 48 Stunden bildete sich bei allen Probekörpern Apatit. Dies zeigt die gute Bioaktivität der erfindungsgemäßen Glaskeramiken und ihre besondere Eignung zur Verwendung als Dentalmaterial oder zur Förderung des Knochenwachstums.

Zellkulturuntersuchungen mit SAOS-2-Zellen (menschliche Tumorzellen) zeigten, dass bereits nach einer Stunde Reaktionszeit die Zelle beginnt, sich auf der Oberfläche der erfindungsgemäßen Glaskeramik zu adhärieren. Nach 24 Stunden ist die ursprünglich kugelförmige Zelle fast vollständig verflacht und haftet fest auf der Oberfläche. Charakteristische Merkmale für schnelle und vorteilhafte bioaktive Prozesse der Knochenregeneration sind die bei Zellkulturtests beobachtete Zellvermehrung (Proliferation) und Zelldifferentiation. Es wurde überraschend festgestellt, dass dieser Prozess bei der erfindungsgemäßen Rhenanit-Glaskeramik exponentiell verläuft, eine sehr vorteilhafte Eigenschaft bei Biowerkstoffen. Ein Zellsterben wurde nicht beobachtet. Weiterhin wurde in den Zellkulturtests und den Versuchen in simulierter Körperflüssigkeit (SBF) die Bildung von Hydroxylapatit festgestellt. Letzteres trifft für monolithische und poröse Werkstoffe gleichermaßen zu. Die gebildeten Apatite sind nadelförmig und lagern sich zu kugligen Aggregaten zusammen. Nach sieben Tagen wurde eine mehr als 0,5 µm dicke Schicht aus Hydroxylapatit auf der Oberfläche der bioaktiven Werkstoffe gebildet. Neben dem Apatit tritt als Nebenkristallphase noch Octacalciumphosphat, eine Vorstufe des Apatits, auf.

**Tabelle 2:**

| **Nummer** | **8** | **10** | **13** | **14** | **15** |
|---|---|---|---|---|---|
| Schmelz temperatur | 1400 °C | 1450 °C | 1550 °C | 1600 °C | 1400 °C |
| Schmelz dauer | 1,5 h | 1, 0 h | 1, 5 h | 1,0 h | 1,5 h |
| Aussehen | glasig farblos trans parent | glasig leicht ent mischt | weiß krista llin | weiß krista llin | glasig farblos trans parent |
| Wärmebe handlung | 2 h / 750°C | 8 h / 750°C | keine | keine und | 750 °C/8 h 900 °C/1h |
| T_{g} [°C] | 500 | 540 | kein | kein | 500 |
| T Kristallisation [°C] | 660 | 640 / 750 | 670 | 580 / 610 | ** |
| Kristall phase | β-NaCaPO₄ | β-NaCaPO₄ Na₂CaSi ₃O₈ | β-NaCaPO₄ | β-NaCaPO₄ KNaCa₂ (P O₄)₂ | β-NaCaPO₄ |
| Anteil an Rhenanit [Gew.-%] | 13 | 22 | 45 | 47^{*} | ca. 5 |
| Bildung von Apatit in SBF nach | 24 h | 24 h | 48 h | 24 h | 24 h |

| | | | | | |
|---|---|---|---|---|---|
| * bei der Glaskeramik Nr. 14 betrug der Anteil an β-NaCa(PO₄) und KNaCa₂(PO₄)₂ zusammen 47 Gew.-% ** Identifizierung der Kristallisation mittels REM, Kristallitgrösse von 20 bis 100 nm | | | | | |

## Patentansprüche

1. Bioaktive Rhenanit-Glaskeramik mit kristalliner Phase und Glasphase, **dadurch gekennzeichnet, dass** die kristalline Phase Rhenanit enthält und die Glaskeramik die folgenden Komponenten
| Komponenten | Anteil (Gew.-%) |
|---|---|
| SiO₂ | 29,5 bis 70,0 |
| CaO | 5,5 bis 23,0 |
| Na₂O | 6,0 bis 27,5 |
| P₂O₅ | 2,0 bis 23,5 |
| F | 0 bis 1,5 |
enthält und im Wesentlichen frei von Al₂O₃ ist.

2. Glaskeramik nach Anspruch 1, die zwischen 4 und 50 Gew.-% Rhenanit enthält.

3. Glaskeramik nach Anspruch 1 oder 2, die zwischen 10 und 50 Gew.-% Rhenanit enthält.

4. Glaskeramik nach einem der Ansprüche 1 bis 3, die die folgenden Komponenten unabhängig voneinander in folgenden Mengen enthält:
| Komponenten | Anteil (Gew.-%) |
|---|---|
| SiO₂ | 29,5 bis 65,5 |
| CaO | 6,0 bis 23,0 |
| | (insbesondere 11,0 bis 23,0) |
| Na₂O | 7,0 bis 25,5 |
| P₂O₅ | 3,0 bis 23,5 |
| | (insbesondere 5,5 bis 23,5) |
| F | 0,5 bis 1,2 |

5. Glaskeramik nach einem der Ansprüche 1 bis 4, die die folgenden Komponenten unabhängig voneinander in folgenden Mengen enthält:
| Komponenten | Anteil (Gew.-%) |
|---|---|
| SiO₂ | 35,0 bis 60,0 |
| CaO | 15,0 bis 23,0 |
| Na₂O | 9,0 bis 25,5 |
| | (insbesondere 7,0 bis 18,0) |
| P₂O₅ | 10,0 bis 23,5 |
| | (insbesondere 10,0 bis 20,0) |
| F | 0,5 bis 1,2 |

6. Glaskeramik nach einem der Ansprüche 1 bis 5, bei der das Gewichtsverhältnis von Na₂O : CaO von 1,0 bis 2,1 und das Gewichtsverhältnis von CaO : P₂O₅ von 0,9 bis 2,2 beträgt.

7. Glaskeramik nach einem der Ansprüche 1 bis 5, bei der das Gewichtsverhältnis von Na₂O : CaO von 0,8 bis 2,0 und das Gewichtsverhältnis von CaO : P₂O₅ von 0,9 bis 2,2 beträgt.

8. Glaskeramik nach einem der Ansprüche 1 bis 7, die zusätzlich mindestens eine der folgenden Zusatz-Komponenten enthält:
| Zusatz-Komponenten | Anteil (Gew.-%) |
|---|---|
| R^{(I)}₂O | 0 bis 15,0 |
| R^{(II)}O | 0 bis 4,0 |
| R^{(III)}₂O₃ | 0 bis 10,0 |
| R^{(IV)}O₂ | 0 bis 10,0 insbesondere bis 1,0 |
| Hal | 0 bis 2,0 |
wobei
R^{(I)} für ein einwertiges Kation, insbesondere K oder Ag,
R^{(II)} für ein zweiwertiges Kation, insbesondere Zn,
R^{(III)} für ein dreiwertiges Kation, insbesondere B, Nb, Ta,
Y, La oder ein Lanthanid,
R^{(IV)} für ein vierwertiges Kation, insbesondere Ti, und
Hal für ein Halogenidion, insbesondere Br oder I
steht.

9. Glaskeramik nach einem der Ansprüche 1 bis 8, bei der die kristalline Phase zusätzlich mindestens eine der folgenden kristallinen Komponenten enthält: Natrium-Calcium-Silicat, Apatit, Natrium-Phosphat, Natrium-Calcium-Phosphat und Natrium-Kalium-Calcium-Phosphat.

10. Glaskeramik nach einem der Ansprüche 1 bis 9, bei der die Rhenanitkristalle im Maximum 10 µm groß sind.

11. Glaskeramik nach einem der Ansprüche 1 bis 10, bei der die Rhenanitkristalle eine mittlere Größe (Zahlenmittel) von 0,01 bis 5,0 µm aufweisen.

12. Glaskeramik nach Anspruch 11, bei der die Rhenanitkristalle eine mittlere Größe (Zahlenmittel) von 0,15 bis 2,5 µm, insbesondere 0,5 bis 2,5 µm aufweisen.

13. Verfahren zur Herstellung einer Glaskeramik gemäß einem der Ansprüche 1 bis 12, bei dem:
a) ein Ausgangsglas, das die Komponenten der Rhenanit-Glaskeramik gemäß Anspruch 1 oder 8 enthält, bei Temperaturen von 1200 °C bis 1650 °C erschmolzen wird,
b) die Glasschmelze aus a) abgekühlt wird,
c) gegebenenfalls das abgekühlte Glas aus b) bei Temperaturen von 600 bis 1000, insbesondere 600 °C bis 980 °C, für eine Dauer von 10 Minuten bis zu 10 Stunden, insbesondere bis zu 8 Stunden wärmebehandelt
und
d) gegebenenfalls die Glaskeramik, die aus b) oder c) resultiert, zu einem Pulver mit einer Korngröße von 100 nm bis 100 µm, insbesondere 1 bis 50 µm zerkleinert.

14. Formkörper, der eine Glaskeramik gemäß einem der Ansprüche 1 bis 12 enthält.

15. Formkörper, der aus einer Glaskeramik gemäß einem der Ansprüche 1 bis 12 besteht.

16. Verwendung einer Glaskeramik gemäß einem der Ansprüche 1 bis 12 oder eines Formkörpers gemäß einem der Ansprüche 14 oder 15 als Material zum Wiederaufbau oder Ersatz von Knochen oder natürlichem Zahnmaterial, oder zur Förderung des Knochenwachstums.

17. Bioaktives Kompositmaterial, das die Glaskeramik gemäß einem der Ansprüche 1 bis 12 und eine organische Verbindung enthält.

## Claims

1. Bioactive rhenanite glass ceramic having a crystalline phase and a glass phase, wherein the crystalline phase comprises rhenanite and the glass ceramic comprises the following components
| Components | Amount (wt.%) |
|---|---|
| SiO₂ | 29.5 to 70.0 |
| CaO | 5.5 to 23.0 |
| Na₂O | 6.0 to 27.5 |
| P₂O₅ | 2.0 to 23.5 |
| F | 0 to 1.5 |
and is essentially free of Al₂O₃.

2. Glass ceramic according to claim 1, comprising between 4 and 50 wt.% rhenanite.

3. Glass ceramic according to claim 1 or 2, comprising between 10 and 50 wt.% rhenanite.

4. Glass ceramic according to one of claims 1 to 3, comprising, independently of each other, the following components:
| Components | Amount (wt.%) |
|---|---|
| SiO₂ | 29.5 to 65.5 |
| CaO | 6.0 to 23.0 (in particular 11.0 to 23.0) |
| Na₂O | 7.0 to 25.5 |
| P₂O₅ | 3.0 to 23.5 (in particular 5.5 to 23.5) |
| F | 0.5 to 1.2. |

5. Glass ceramic according to one of claims 1 to 4, comprising, independently of each other, the following components:
| Components | Amount (wt.%) |
|---|---|
| SiO₂ | 35.0 to 60.0 |
| CaO | 15.0 to 23.0 |
| Na₂O | 9.0 to 25.5 (in particular 7.0 to 18.0) |
| P₂O₅ | 10.0 to 23.5 (in particular 10.0 to 20.0) |
| F | 0.5 to 1.2. |

6. Glass ceramic according to one of claims 1 to 5, in which the weight ratio of Na₂O: CaO is from 1.0 to 2.1 and the weight ratio of CaO: P₂O₅ is from 0.9 to 2.2.

7. Glass ceramic according to one of claims 1 to 5, in which the weight ratio of Na₂O: CaO is from 0.8 to 2.0 and the weight ratio of CaO: P₂O₅ is from 0.9 to 2.2.

8. Glass ceramic according to one of claims 1 to 7, additionally comprising at least one of the following added components:
| Added components | Amount (wt.%) |
|---|---|
| R^{(I)}₂O | 0 to 15.0 |
| R^{(II)}O | 0 to 4.0 |
| R^{(III)}₂O₃ | 0 to 10.0 |
| R^{(IV)}O₂ | 0 to 10.0 in particular up to 1.0 |
| Hal | 0 to 2.0 |
wherein
R^{(I)} represents a monovalent cation, in particular K or Ag
R^{(II)} represents a divalent cation, in particular Zn
R^{(III)} represents a trivalent cation, in particular B, Nb, Ta, Y,
La or a lanthanide,
R^{(IV)} represents a tetravalent cation, in particular Ti and
Hal represents a halide ion, in particular Br or 1.

9. Glass ceramic according to one of claims 1 to 8, in which the crystalline phase further comprises at least one of the following crystalline components: sodium calcium silicate, apatite, sodium phosphate, sodium calcium phosphate and sodium potassium calcium phosphate.

10. Glass ceramic according to one of claims 1 to 9, in which the rhenanite crystals are at most 10 µm in size.

11. Glass ceramic according to one of claims 1 to 10, in which the rhenanite crystals have an average size (number average) of 0.01 to 5.0 µm.

12. Glass ceramic according to claim 11, in which the rhenanite crystals have an average size (number average) of 0.15 to 2.5 µm, particularly 0.5 to 2.5 µm.

13. Process for manufacturing a glass ceramic according to one of claims 1 to 12, in which:
a) a starting glass comprising the components of the rhenanite glass ceramic according to claim 1 or 8 is melted at temperatures of 1200 °C to 1650 °C,
b) the molten glass from a) is cooled,
c) the cooled glass from b) is optionally heat treated at temperatures of 600 to 1000, in particular 600 °C to 980 °C, for a period of 10 minutes to 10 hours, in particular for up to 8 hours, and
d) the glass ceramic resulting from b) or c) is optionally comminuted to a powder having a particle size of 100 nm to 100 µm, in particular 1 to 50 µm.

14. Moulded article comprising a glass ceramic according to one of claims 1 to 12.

15. Moulded article consisting of a glass ceramic according to one of claims 1 to 12.

16. Use of a glass ceramic according to one of claims 1 to 12 or a moulded article according to one of claims 14 or 15 as a reconstructive material or replacement of bones or natural tooth material, or for promoting bone growth.

17. Bioactive composite material comprising the glass ceramic according to one of claims 1 to 12 and an organic compound.

## Revendications

1. Vitrocéramique bioactive à base de rhénanite, comportant une phase cristalline et une phase vitreuse, **caractérisée en ce que** la phase cristalline contient de la rhénanite et **en ce que** cette vitrocéramique contient les composants suivants :
| Composant | Proportion (% pds) |
|---|---|
| SiO₂ | 29,5 à 70,0 |
| CaO | 5,5 à 23,0 |
| Na₂O | 6,0 à 27,5 |
| P₂O₅ | 2,0 à 23,5 |
| F | 0 à 1,5 |
et ne contient pratiquement pas d'alumine Al₂O₃.

2. Vitrocéramique conforme à la revendication 1, qui contient de 4 à 50 % en poids de rhénanite.

3. Vitrocéramique conforme à la revendication 1 ou 2, qui contient de 10 à 50 % en poids de rhénanite.

4. Vitrocéramique conforme à l'une des revendications 1 à 3, qui contient les composants suivants, indépendamment les uns des autres, en les proportions indiquées ci-dessous :
| Composant | Proportion (% pds) |
|---|---|
| SiO₂ | 29,5 à 65,5 |
| CaO | 6,0 à 23,0 |
| | (en particulier 11,0 à 23,0) |
| Na₂O | 7,0 à 25,5 |
| P₂O₅ | 3,0 à 23,5 |
| | (en particulier 5,5 à 23,5) |
| F | 0,5 à 1,2 |

5. Vitrocéramique conforme à l'une des revendications 1 à 4, qui contient les composants suivants, indépendamment les uns des autres, en les proportions indiquées ci-dessous :
| Composant | Proportion (% pds) |
|---|---|
| SiO₂ | 35,0 à 60,0 |
| CaO | 15,0 à 23,0 |
| Na₂O | 9,0 à 25,5 |
| | (en particulier 7,0 à 18,0) |
| P₂O₅ | 10,0 à 23,5 |
| | (en particulier 10,0 à 20,0) |
| F | 0,5 à 1,2 |

6. Vitrocéramique conforme à l'une des revendications 1 à 5, dans laquelle le rapport pondéral Na₂O/CaO vaut de 1,0 à 2,1 et le rapport pondéral CaO/P₂O₅ vaut de 0,9 à 2,2.

7. Vitrocéramique conforme à l'une des revendications 1 à 5, dans laquelle le rapport pondéral Na₂O/CaO vaut de 0,8 à 2,0 et le rapport pondéral CaO/P₂O₅ vaut de 0,9 à 2,2.

8. Vitrocéramique conforme à l'une des revendications 1 à 7, qui contient en outre au moins l'un des adjuvants suivants :
| Adjuvant | Proportion (% pds) |
|---|---|
| R^{(I)}₂O | 0 à 15,0 |
| R^{(II)}O | 0 à 4,0 |
| R^{(III)}₂O₃ | 0 à 10,0 |
| R^{(IV)}O₂ | 0 à 10,0 |
| | (en particulier à 1,0) |
| Hal | 0 à 2,0 |
étant entendu que :
R^{(I)} représente un cation monovalent, en particulier de potassium ou d'argent,
R^{(II)} représente un cation divalent, en particulier de zinc,
R^{(III)} représente un cation trivalent, en particulier de bore, de niobium, de tantale, d'yttrium, de lanthane ou d'un lanthanide,
R^{(IV)} représente un cation tétravalent, en particulier de titane,
et Hal représente un ion halogénure, en particulier de brome ou d'iode.

9. Vitrocéramique conforme à l'une des revendications 1 à 8, dans laquelle la phase cristalline contient en outre au moins l'un des composants cristallins suivants : silicate de sodium et calcium, apatite, phosphate de sodium, phosphate de sodium et calcium, et phosphate de sodium, potassium et calcium.

10. Vitrocéramique conforme à l'une des revendications 1 à 9, dans laquelle les cristaux de rhénanite présentent une taille maximale de 10 µm.

11. Vitrocéramique conforme à l'une des revendications 1 à 10, dans laquelle les cristaux de rhénanite présentent une taille moyenne en nombre de 0,01 à 5,0 µm.

12. Vitrocéramique conforme à la revendication 11, dans laquelle les cristaux de rhénanite présentent une taille moyenne en nombre de 0,15 à 2,5 µm, et en particulier de 0,5 à 2,5 µm.

13. Procédé de fabrication d'une vitrocéramique conforme à l'une des revendications 1 à 12, dans lequel :
a) on fait fondre, à une température de 1200 à 1650 °C, un verre de départ qui contient les composants de vitrocéramique à base de rhénanite indiqués dans la revendication 1 ou 8 ;
b) on fait refroidir la masse de verre fondu issue de l'étape (a) ;
c) en option, on soumet le verre refroidi issu de l'étape (b) à un traitement thermique, à une température de 600 à 1000 °C et en particulier de 600 à 980 °C, durant un laps de temps de 10 minutes à 10 heures et en particulier à 8 heures ;
d) et en option, on broie la vitrocéramique résultant de l'étape (b) ou (c) pour en faire une poudre dont les particules ont une taille de 100 nm à 100 µm et en particulier de 1 à 50 µm.

14. Corps façonné, contenant une vitrocéramique conforme à l'une des revendications 1 à 12.

15. Corps façonné, constitué d'une vitrocéramique conforme à l'une des revendications 1 à 12.

16. Utilisation d'une vitrocéramique conforme à l'une des revendications 1 à 12, ou d'un corps façonné conforme à l'une des revendications 14 et 15, en tant que matériau pour la reconstitution ou le remplacement d'os ou de matériau dentaire naturel, ou pour accélérer la croissance osseuse.

17. Matériau composite bioactif, qui contient une vitrocéramique conforme à l'une des revendications 1 à 12 et un composé organique.
